# EUROPEAN PATENT SPECIFICATION

(11) **EP 3 551 293 B1**
(45) Date of publication and mention of the grant of the patent: **09.02.2022**
(21) Application number: 17880407.6
(22) Date of filing: 05.12.2017
(51) Int. Cl.: A61P 31/04, C12M 1/34, C12Q 1/02, C12Q 1/04, C12Q 1/06, C12Q 1/6806

(54) **RNASE FOR IMPROVED MICROBIAL DETECTION AND ANTIMICROBIAL SUSCEPTIBILITY TESTING**
RNASE FÜR VERBESSERTE MIKROBENDETEKTION UND TESTUNG DER ANTIMIKROBIELLEN SUSZEPTIBILITÄT
RNASE POUR UNE DÉTECTION MICROBIENNE ET UNE ANALYSE DE LA SENSIBILITÉ ANTIMICROBIENNE AMÉLIORÉES

(30) Priority: 06.12.2016 US 201662430785 P
(43) Date of publication of application: 16.10.2019
(73) Proprietor: MicrobeDx, Inc., Novato, CA 94945 (US); The Regents of the University of California, Oakland, CA 94607 (US)
(72) Inventor: HAAKE, David, A., Culver City, CA 90230 (US); GUSSIN, Daniel, Encinitas, CA 92024 (US); MONTI, Gabriel K., Cypress, CA 90630 (US); CHURCHILL, Bernard M., Los Angeles, CA (US); HALFORD, Colin W., Los Angeles, CA 90095-1406 (US)
(74) Representative: Novitas Patent AB
(86) International application number: PCT/US2017/064774
(87) International publication number: WO 2018/111630

(56) References cited:
- WO-A1-2013/123440
- WO-A1-2013/123440
- WO-A1-2013/166460
- WO-A1-2015/013324
- WO-A2-2016/085632
- US-A1- 2013 157 876
- US-A1- 2015 104 789
- US-A1- 2015 250 184
- US-A1- 2016 160 268
- SKERLOS S J ET AL: "EXPEDITIONS IDENTIFICATION AND QUANTIFICATION OF MYCOBACTERIA SPECIES IN METALWORKING FLUIDS USING PEPTIDE NUCLEIC ACID PROBES", JOURNAL OF MANUFACTURING SYSTEMS, SOCIETY OF MANUFACTURING ENGINEERS, DEARBORN, MI, US, vol. 22, no. 2, 1 January 2003 (2003-01-01), pages 136-147, XP008071139, ISSN: 0278-6125
- C. HALFORD ET AL: "Rapid Antimicrobial Susceptibility Testing by Sensitive Detection of Precursor rRNA Using a Novel Electrochemical Biosensing Platform", ANTIMICROBIAL AGENTS AND CHEMOTHERAPY, vol. 57, no. 2, 10 December 2012 (2012-12-10), pages 936-943, XP055550508, US ISSN: 0066-4804, DOI: 10.1128/AAC.00615-12
- HALFORD et al.: "Rapid Antimicrobial Susceptibility Testing by Sensitive Detection of Precursor rRNA Using a Novel Electrochemical Biosensing Platform", Antimicrobial Agents and Chemotherapy, vol. 57, no. 2, 10 December 2012 (2012-12-10), pages 936-943, XP055550508,
- ROGACS et al.: "Bacterial RNA extraction and purification from whole human blood using isotachopnoresls", Analytical Chemistry, vol. 84, no. 14, 29 June 2012 (2012-06-29) , pages 5858-5863, XP055550542,
- SONG et al.: "Antibiotic stress-induced modulation of the endoribonucleolytic activity of RNase III and RNase G confers resistance to aminoglycoside antibiotics in Escherichia coli", Nucleic Acids Research, vol. 42, no. 7, 30 January 2014 (2014-01-30), pages 4669-4681, XP055550558,

## Description

### ACKNOWLEDGEMENT OF GOVERNMENT SUPPORT

This invention was made with government support under Grant No. Al109889, awarded by the National Institutes of Health. The government has certain rights in the invention.

### BACKGROUND OF THE INVENTION

There is an urgent need for the development of rapid, convenient, and accurate methods for detection and identification of antibiotic-resistant bacterial pathogens in clinical specimens to guide diagnosis and treatment of infectious diseases. The best outcomes are achieved when antibiotic therapy is based on identification of the pathogen and its antibiotic sensitivity. Out of concerns regarding the seriousness of the disease, therapy is often started before this information is available. The effectiveness of individual antibiotics varies with the resistance of the bacterial pathogen to the antibiotic. Therapeutic outcomes can be significantly improved by the availability of a rapid assay for antibiotic susceptibility.

Ribosomal RNA is an excellent target molecule for pathogen detection systems because of its abundance in the bacterial cell and because of the accessibility of species-specific signature sequences to probe hybridization. When combined with sensitive surface chemistry methods to minimize nonspecific background signals, such rRNA probe hybridization sensors are able to detect as few as 100 bacteria per ml. Estimations of bacterial density are possible because, within the dynamic range of the assay, there is a log-log correlation between the concentration of target rRNA molecules in the bacterial lysate and the assay signal. The accuracy of bacterial quantitation methods based on rRNA detection is mitigated by variations in the number of rRNA molecules per cell depending on the microbial species and its growth phase. In *E. coli,* the rRNA copy number per cell has been estimated to vary from as high as ~100,000 during log phase to less than 5,000 during stationary phase (Halford, C., et al., Antimicrob. Agents Chemother. 57(2):936-43 (2013). PMID: 23229486). WO 2013/166460 A1 (UNIV CALIFORNIA [US] ET AL.) discloses a method to determine if a sample of bacteria is susceptible to an antibiotic, by contacting 2 specimens of the bacteria with oligonucleotide probes that hybridise with the rRNA of these bacteria, whereby one of the samples is inoculated with an antibiotic such as beta lactam and the relative amounts of hybridisation are used to determine and identifying the sample as susceptible if the amount of target detected is smaller for the sample with the antibiotic.

There remains a need for improved methods of detecting bacterial pathogens and bacterial susceptibility to antibiotic treatment, and methods of reducing background for samples that contain free rRNA to improve sensitivity of methods for measurement of rRNA.

### SUMMARY OF THE INVENTION

The present invention relates generally to materials and methods for detection of bacteria, and for testing and determination of antibiotic susceptibility of bacteria in specimens of bodily fluid and other samples. The invention also relates to materials and methods for monitoring the physiological response of bacteria to antimicrobial agents, and for reducing background and increasing sensitivity of assays that involve the detection and/or measurement of RNA, such as rRNA.

### BRIEF DESCRIPTION OF THE FIGURES

**FIG. 1** is a graph which depicts the effect of RNase on critical limit (Lc) and limit of detection (Ld).
**FIG. 2** is a graph showing the effect of RNase on rRNA response of resistant (red arrows) and susceptible (black arrows) to antibiotics namely ampicillin (Amp), cefazolin (Cef), ceftriaxone (Ctrx), and Fosfomycin (Fom). P-values are shown underneath each antibiotic.
**FIG. 3** depicts graph showing the effect of RNase on rRNA response of resistant (red arrows) and susceptible (black arrows) to Fosfomycin at 128 µg/ml, 64 µg/ml and 32 µg/ml. P-values are also shown above each Fosfomycin concentration used.
**FIG. 4** depicts a comparison of antimicrobial susceptibility tests with and without 1 µg/ml RNase for *Escherichia coli* treated with Ampicillin (Amp).
**FIG. 5** depicts a comparison of antimicrobial susceptibility tests with and without 1 µg/ml RNase for *Escherichia coli* treated with Cefazolin (Cef).
**FIG. 6** depicts depicts a comparison of antimicrobial susceptibility tests with and without 1 µg/ml RNase for *Escherichia coli* treated with Cefepime (Cpm).
**FIG. 7** depicts depicts a comparison of antimicrobial susceptibility tests with and without 1 µg/ml RNase for *Escherichia coli* treated with Ceftriaxone (Ctrx).
**FIG. 8** depicts a comparison of antimicrobial susceptibility tests with and without 1 µg/ml RNase for *K. pneumoniae* treated with Ceftriaxone (Ctrx).

### DETAILED DESCRIPTION OF THE INVENTION

The invention is based on two surprising and counterintuitive discoveries: First, that an enzyme (RNase) that degrades the target analyte (rRNA) can be used to assist in the detection of that analyte. In contrast, conventional practice is to eliminate RNases from assays for RNA. The invention provides new methods for use in tests that target rRNA to detect, identify, and perform antimicrobial susceptibility testing (AST) on microbes or microorganisms. In a typical assay, levels of rRNA are measured by sample lysis and hybridization of rRNA with capture and detector probes. In one embodiment, the detector probe is linked to a signaling molecule with enzymatic (e.g., Horseradish peroxidase) or optical (e.g., fluorescence, bioluminescence) features. Samples containing free rRNA not from living cells increases background, lowers the signal-to-noise ratio and increases the limit of detection. A lower limit of detection improves the sensitivity of diagnostic tests designed to detect microbes. Sensitivity is also important for phenotypic AST based on the rRNA response to antibiotics.

The second counterintuitive discovery is that an enzyme (RNase) that degrades the target analyte (rRNA) can be used to more rapidly determine the susceptibility of bacteria to an antibiotic where that analyte is utilized as a surrogate marker for the phenotypic response to antibiotics. Bacterial suspensions are inoculated into growth medium with and without antibiotics, followed by incubation at 37°C. At the conclusion o f the incubation period, comparison of rRNA levels with and without antibiotics enables determination of whether the bacterial isolate is susceptible or resistant to the tested antibiotic. In the case of antibiotics that act on the cell wall of bacteria, such as beta-lactam antibiotics and fosfomycin, RNase degrades rRNA of bacteria exposed to antibiotics to which they are susceptible. Degradation of rRNA in an AST assists in differentiating susceptible from resistant bacteria and accelerates the time to results that enable therapy with antibiotics to which the patient's microbes are susceptible.

RNase can also be used as a general means of reducing background and improving sensitivity when measuring rRNA directly, or as an indicator of antimicrobial susceptibility, or other assays affected by the presence of free rRNA.

In some embodiments, the microorganism is a prokaryote. In some embodiments, the prokaryote is a Gram-negative bacteria. In some embodiments, the prokaryote is a Gram-positive bacteria.

In some embodiments, the microorganism is fungal (e.g., candida). In some embodiments, at least one antimicrobial agent is an antifungal agent. In some embodiments, the antifungal agent is a fungicide. In some embodiments, the antifungal agent is a fungistatic. In some embodiments, the antifungal agent is a triazole antifungal agent. In some embodiments, the triazole antifungal agent is selected from the group of fluconazole and itraconazole.

### Definitions

All scientific and technical terms used in this application have meanings commonly used in the art unless otherwise specified. As used in this application, the following words or phrases have the meanings specified.

As used herein, "cell wall active antibiotics" refers to antibacterial agents that target bacterial cell walls or cell membranes. In some embodiments, the cell wall active antibiotics are β-lactams, which include penicillins, cephalosporins, carbapenems and monobactams. In some embodiments, the cell wall active antibiotics are glycopeptides or fosfomycin. Membrane-active antibiotic agents include daptomycin, colistin, polymyxin B, monensin, and salinomycin.

The term "nudeic acid" or "polynucleotide" or "oligonucleotide" refers to a sequence of nucleotides, a deoxyribonucleotide or ribonucleotide polymer in either single- or double-stranded form, and unless otherwise limited, encompasses known analogs of natural nucleotides that hybridize to nucleic acids in a manner similar to naturally occurring nucleotides.

The term "probe," as used herein, means an oligonucleotide designed to hybridize with an rRNA target region. In a probe pair, one probe is complementary to nucleotides present on the rRNA target and another probe is complementary to nucleotides on an adjacent rRNA target region. A probe can hybridize to a rRNA target region of at least about 11 nucleotides, and preferably, at least about 16 nucleotides and no more than about 35 nucleotides in length. The probe itself can be longer than the primer-target hybridization region, up to 50 nucleotides in length. The total length of the hybridization region bound by the probe pair is at least 22 nucleotides and no more than 70 nucleotides in length. Typically, a hybridization region has at least about 80% sequence identity, preferably at least about 90% sequence identity with a target polynucleotide to which the probe hybridizes. The "probe" can be an oligonucleotide, naturally or synthetically produced, via recombinant methods or by PCR amplification, that hybridizes to at least part of another oligonucleotide of interest. A probe can be single-stranded or double-stranded. Examples of probe pairs are universal probe pairs that detect rRNA from all microbes, group-specific probe pairs that detect rRNA common to a family or microbial genus, and species-specific probe pairs that detect rRNA only from a single species. Typically the probe pair consists of a capture probe and a detector probe where the capture probe anchors the target molecule to a surface or bead and the detector probe enables a detection mechanism such as electrochemical or optical detection.

As used herein, the term "active fragment" refers to a substantial portion of an oligonucleotide that is capable of performing the same function of specifically hybridizing to a target polynucleotide.

As used herein, "hybridizes," "hybridizing," and "hybridization" means that the oligonucleotide forms a noncovalent interaction with the target DNA molecule under standard conditions. Standard hybridizing conditions are those conditions that allow an oligonucleotide probe or primer to hybridize to a target DNA molecule. Such conditions are readily determined for an oligonucleotide probe or primer and the target DNA molecule using techniques well known to those skilled in the art. The nucleotide sequence of a target polynucleotide is generally a sequence complementary (as defined below) to the oligonucleotide primer or probe. The hybridizing oligonucleotide may contain nonhybridizing nucleotides that do not interfere with forming the noncovalent interaction. The nonhybridizing nucleotides of an oligonucleotide primer or probe may be located at an end of the hybridizing oligonucleotide or within the hybridizing oligonucleotide. Thus, an oligonucleotide probe or primer does not have to be complementary to all the nucleotides of the target sequence as long as there is hybridization under standard hybridization conditions. Hybridization can be defined as the interaction between a probe and its rRNA target in a buffer and temperature such as 1M phosphate buffer at 25°C with a sufficient strigency to prevent non-specific hybridization.

The term "complement" and "complementary" as used herein, refers to the ability of two nucleotide molecules to base pair with each other, where an adenine on one DNA molecule will base pair to a guanine on a second DNA molecule and a cytosine on one DNA molecule will base pair to a thymine on a second DNA molecule. Two DNA molecules are complementary to each other when a nucleotide sequence in one DNA molecule can base pair with a nucleotide sequence in a second DNA molecule. For instance, the two DNA molecules 5'-ATGC and 5'-GCAT are complementary, and the complement of the DNA molecule 5'-ATGC is 5'-GCAT. The term complement and complementary also encompasses two DNA molecules where one DNA molecule contains at least one nucleotide that will not base pair to at least one nucleotide present on a second DNA molecule. For instance, the third nucleotide of each of the two DNA molecules 5'-ATTGC and 5'-GCTAT will not base pair, but these two DNA molecules are complementary as defined herein. Typically, two DNA molecules are complementary if they hybridize under the standard conditions referred to above. Typically, two nucleotide molecules are complementary if they have at least about 80% sequence complementarity, preferably at least about 90% sequence complementarity. The probe may have 100% sequence complementarity with the target sequence. Complementarity can involve the use of synthetic nucleotides.

Probes which used in the present methods can be Eubacterial/Universal Gram Negative:
SEQ ID NO: 1 - 5'-GTTACGACTTCACCCCAG-3'
SEQ ID NO: 2 - 5'-CATAATCAATTTCAACTTTCTACT-3'
SEQ ID No: 3 - 5'-GTTACGACTTCACCCCAGCATAATCAATTTCAACTTTCTACT-3'
SEQ ID No: 4 - 5'-GTTCCCCTACGGTTACCTT-3'

As used herein, "a" or "an" means at least one, unless clearly indicated otherwise.

As used herein, to "prevent" or "protect against" a condition or disease means to hinder, reduce or delay the onset or progression of the condition or disease.

As used herein, the term "isolated" means that a naturally occurring DNA fragment, DNA molecule, coding sequence, or oligonucleotide is removed from its natural environment, or is a synthetic molecule or cloned product. Preferably, the DNA fragment, DNA molecule, coding sequence, or oligonucleotide is purified, i.e., essentially free from any other DNA fragment, DNA molecule, coding sequence, or oligonucleotide and associated cellular products or other impurities.

### Methods of the Invention

The invention provides, among other innovations, methods for assaying rRNA, for determining susceptibility to antimicrobial agents, and for improving the sensitivity of such assays.

In one embodiment, the invention provides a method for determining whether a sample of bacteria is susceptible to an antibiotic agent.

In one embodiment, the method comprises: (a) inoculating a specimen obtained from the sample into a growth medium in the presence of an antibiotic agent, wherein the growth medium comprises an RNase that hydrolyzes ribosomal RNA (rRNA); (b) inoculating a specimen obtained from the sample into a growth medium in the absence of the antibiotic agent, wherein the growth medium comprises an RNase that is enzymatically active against rRNA. The method further comprises (c) measuring the relative amounts of rRNA in the specimens of (a) and (b); and identifying the sample as susceptible to antibiotic treatment if the amount of rRNA measured in step (a) is reduced relative to the amount of rRNA measured in step (b).

In one embodiment, the method comprises: (a) inoculating a specimen obtained from the sample into a growth medium in the presence a cell wall active antibiotic agent, wherein the growth medium comprises an RNase that hydrolyzes ribosomal RNA (rRNA); (b) inoculating a specimen obtained from the sample into a growth medium in the absence of the antibiotic agent, wherein the growth medium comprises an RNase that is enzymatically active against rRNA. The method further comprises (c) measuring the relative amounts of rRNA in the specimens of (a) and (b); and identifying the sample as susceptible to antibiotic treatment if the amount of rRNA measured in step (a) is reduced relative to the amount of rRNA measured in step (b).

A wide range of RNase concentrations may be used in this method, from 0.01 to 10 micrograms RNase per milliliter growth medium.

In some embodiments, the RNase concentration used in this method is 0.02, 0.03, 0.04, 0.05, 0.06, 0.07, 0.08, 0.09, 0.1, 0.2, 0.3, 0.4, 0.5, 0.6, 0.7, 0.8, 0.9, 1, 1.5, 2, 2.5, 3, 3.5, 4, 4.5, 5, 5.5, 6, 6.5, 7, 7.5, 8, 8.5, 9 or 9.5 micrograms RNase per milliliter growth medium.

While all concentrations tested in the aforementioned range were effective at scavenging free rRNA, 1 microgram RNase per milliliter growth medium provided the greatest enhancement of relative rRNA difference between specimens (a) and (b).

In one embodiment, the RNase concentration used in the method is 1 microgram RNase per milliliter growth medium.

In some embodiments, the measuring comprises detection of specific hybridization of an oligonucleotide probe to the rRNA. In one representative embodiment, the probe is 10-50 nucleotides in length. In some embodiments, the probe hybridizes to the rRNA over the full length of a target sequence of the rRNA. In one embodiment, the probe is 25-30 nucleotides in length. In some embodiments, the detection comprises the hybridization of two probes, a capture probe and a detector probe. In one embodiment, the combined length of capture and detector probes is 50-60 nucleotides. In one embodiment, the combined length of capture and detector probes is 40-60 nucleotides. In one embodiment, the combined length of capture and detector probes is 30-60 nucleotides. In one embodiment, the combined length of capture and detector probes is 20-60 nucleotides. In one embodiment, the RNase is RNase A. In some embodiments, the RNase is RNase T1, RNase I, RNase VI, or RNase III. RNase VI acts on double stranded (i.e., highly structured) RNA such as rRNA. RNase III specifically acts on pre-rRNA, not mature rRNA. Many RNases have some activity on rRNA, and those skilled in the art can select an appropriate RNase for selected embodiment.

In some embodiments, the method further comprises contacting the sample with a capture probe. In some embodiments, the capture probe comprises a capture sequence comprising a plurality of nucleic acids. In some embodiments, the plurality of nucleic acids comprises one or more deoxyribonucleic acids (DNA). In some embodiments, the plurality of nucleic acids comprises one or more peptide nucleic acids (PNAs). In some embodiments, the plurality of nucleic acids comprises one or more locked nucleic acids (LNAs). In some embodiments, at least a portion of the capture sequence is complementary to at least a portion of a nucleic acid molecule from the microorganism.

In some embodiments, the capture probe comprises 1, 2, 3, 4, 5, 6, 7, 8, 9, 10, 11, 12, 13, 14, 15, 16, 17, 18, 19, 20 or more nucleic acids. In some embodiments, the capture probe comprises at least one of deoxyribonucleic acid (DNA), peptide nucleic acid (PNA), locked nucleic acid (LNA), or any combination thereof. In some embodiments, the capture probe comprises DNA. In some embodiments, the capture probe comprises a plurality of DNA. In some embodiments, the capture probe comprises 1, 2, 3, 4, 5, 6, 7, 8, 9, 10, 11, 12, 13, 14, 15, 16, 17, 18, 19, 20 or more DNA. In some embodiments, the capture probe comprises one or more PNAs. In some embodiments, the capture probe comprises a plurality of PNAs. In some embodiments, the capture probe comprises 1, 2, 3, 4, 5, 6, 7, 8, 9, 10, 11, 12, 13, 14, 15, 16, 17, 18, 19, 20 or more PNAs. In some embodiments, the capture probe comprises one or more LNAs. In some embodiments, the capture probe comprises a plurality of LNAs. In some embodiments, the capture probe comprises 1, 2, 3, 4, 5, 6, 7, 8, 9, 10, 11, 12, 13, 14, 15, 16, 17, 18, 19, 20 or more LNAs. In some embodiments, at least a portion of the capture sequence is complementary to at least a portion of a nucleic acid molecule from the microorganism.

In some embodiments, the detector probe comprises one or more nucleic acids. In some embodiments, the nucleic acids comprise one or more modified oligonucleotides. In some embodiments, the detector probe comprises a plurality of nucleic acids. In some embodiments, the detector probe comprises 1, 2, 3, 4, 5, 6, 7, 8, 9, 10, 11, 12, 13, 14, 15, 16, 17, 18, 19, 20 or more nucleic acids. In some embodiments, the detector probe comprises at least one deoxyribonucleic acid (DNA), peptide nucleic acid (PNA), locked nucleic acid (LNA), or any combination thereof. In some embodiments, the detector probe comprises one or more DNA. In some embodiments, the detector probe comprises a plurality of DNA. In some embodiments, the detector probe comprises 1, 2, 3, 4, 5, 6, 7, 8, 9, 10, 11, 12, 13, 14, 15, 16, 17, 18, 19, 20 or more DNA. In some embodiments, the detector probe comprises one or more PNAs. In some embodiments, the detector probe comprises a plurality of PNAs. In some embodiments, the detector probe comprises 1, 2, 3, 4, 5, 6, 7, 8, 9, 10, 11, 12, 13, 14, 15, 16, 17, 18, 19, 20 or more PNAs. In some embodiments, the detector probe comprises one or more LNAs. In some embodiments, the detector probe comprises a plurality of LNAs. In some embodiments, the detector probe comprises 1, 2, 3, 4, 5, 6, 7, 8, 9, 10, 11, 12, 13, 14, 15, 16, 17, 18, 19, 20 or more LNAs.

In some embodiments, the detector probe comprises a detectable label. In some embodiments, the detectable label is selected from a radionuclide, an enzymatic label, a chemiluminescent label, a hapten and a fluorescent label. In some embodiments, the detectable label is a fluorescent molecule. In some embodiments, the fluorescent molecule is selected from a fluorophore, a cyanine dye, and a near infrared (NIR) dye. In some embodiments, the fluorescent molecule is fluorescein. In some embodiments, the fluorescent molecule is fluorescein isothiocyanate (FITC). In some embodiments, the detectable label is a hapten. In some embodiments, the hapten is selected from DCC, biotin, nitropyrazole, thiazolesulfonamide, benzofurazan, and 2-hydroxyquinoxaline, In some embodiments, the detectable label is biotin.

In some embodiments, the microorganism is a prokaryote. In some embodiments, the prokaryote is a Gram-negative bacteria. In some embodiments, the prokaryote is a Gram-positive bacteria. In some embodiments the prokaryote is a mycobacteria.

In some embodiments, the microorganism is fungi (e.g., candida). In some embodiments, at least one antimicrobial agent is an antifungal agent. In some embodiments, the antifungal agent is a fungicide. In some embodiments, the antifungal agent is a fungistatic. In some embodiments, the antifungal agent is a triazole antifungal agent. In some embodiments, the triazole antifungal agent is selected from the group of fluconazole and itraconazole.

In some embodiments, each inoculate of the plurality of inoculates is in a container. In some embodiments, the container is a well of a tissue culture plate. In some embodiments, the tissue culture plate contains a plurality of wells. In some embodiments, the tissue culture plate contains 6, 12, 24, 48, 96, or more wells.

Examples of rRNA include, but are not limited to, bacterial rRNA and fungal rRNA. Examples of bacterial rRNA include pre-rRNA, 5S rRNA, 16S rRNA, 23S rRNA. Examples of fungal rRNA include pre-rRNA, 5.8S rRNA, 18S rRNA, 25S rRNA. In some embodiments, the antibiotic agent is fosfomycin or a beta lactam antibiotic.

In some embodiments, the method further comprises incubating each inoculate or the plurality of inoculates at 37°C. In some embodiments, the in oculate is incubated for at least 15, 30, 60, 90, 120, 150, 180, 210, 240, 270, 300, 360, 420, or 480 or more minutes. In some embodiments, each inoculate or the plurality of inoculates are incubated for less than 480 minutes, less than 420 minutes, less than 360 minutes, less than 300 minutes, less than 270 minutes, less than 240 minutes, less than 210 minutes, less than 180 minutes, less than 150 minutes, less than 120 minutes, less than 90 minutes, less than 60 minutes or less than 30 minutes. In some embodiments each inoculate or plurality of inoculates are incubated for 120 minutes, 90 minutes or 60 minutes.

The methods disclosed herein comprise the use of one or more antibiotic agents. Use of one or more antimicrobial agents may comprise producing an inoculate comprising a microorganism in a cell culture media containing one or more antibiotic agents. Use of one or more antibiotic agents may comprise obtaining an inoculate comprising a microorganism in a cell culture media containing one or more antibiotic agents. Use of one or more antibiotic agents may comprise exposing a microorganism to one or more antibiotic agents.

In some embodiments, the antibiotic agent is a bactericidal antibiotic. In some embodiments, the antibiotic is a bacteriostatic antibiotic. In some embodiments, the antibiotic is selected from an aminoglycoside antibiotic, a beta-lactam antibiotic, an ansamycin antibiotic, a macrolide antibiotic, a sulfonamide antibiotic, a quinolone antibiotic, an oxazolidinone antibiotic, and a glycopeptide antibiotic.

In some embodiments, the antibiotic is a beta-lactam antibiotic selected from 2-(3-alanyl)clavam, 2-hydroxymethylclavam, 8-epi-thienamycin, acetyl-thienamycin, amoxicillin, amoxicillin sodium, amoxicillin trihydrate, amoxicillin -potassium clavulanate combination, ampicillin, ampicillin sodium, ampicillin trihydrate, ampicillin-sulbactam, apalcillin, aspoxicillin, azidocillin, azlocillin, aztreonam, bacampicillin, biapenem, carbenicillin, carbenicillin disodium, carfecillin, carindacillin, carpetimycin, cefacetril, cefaclor, cefadroxil, cefalexin, cefaloridine, cefalotin, cefamandole, cefamandole, cefapirin, cefatrizine, cefatrizine propylene glycol, cefazedone, cefazolin, cefbuperazone, cefcapene, cefcapene pivoxil hydrochloride, cefdinir, cefditoren, cefditoren pivoxil, cefepime, cefetamet, cefetamet pivoxil, cefixime, cefmenoxime, cefmetazole, cefminox, cefminox, cefmolexin, cefodizime, cefonicid, cefoperazone, ceforanide, cefoselis, cefotaxime, cefotetan, cefotiam, cefoxitin, cefozopran, cefpiramide, cefpirome, cefpodoxime, cefpodoxime proxetil, cefprozil, cefquinome, cefradine, cefroxadine, cefsulodin, ceftazidime, cefteram, cefteram pivoxil, ceftezole, ceftibuten, ceftizoxime, ceftriaxone, cefuroxime, cefuroxime axetil, cephalosporin, cephamycin, chitinovorin, ciclacillin, clavulanic acid, clometocillin, cloxacillin, cycloserine, deoxy pluracidomycin, dicloxacillin, dihydro pluracidomycin, epicillin, epithienamycin, ertapenem, faropenem, flomoxef, flucloxacillin, hetacillin, imipenem, lenampicillin, loracarbef, mecillinam, meropenem, metampicillin, meticillin, mezlocillin, moxalactam, nafcillin, northienamycin, oxacillin, panipenem, penamecillin, penicillin, phenethicillin, piperacillin, tazobactam, pivampicillin, pivcefalexin, pivmecillinam, pivmecillinam hydrochloride, pluracidomycin, propicillin, sarmoxicillin, sulbactam, sulbenicillin, talampicillin, temocillin, terconazole, thienamycin, and ticarcillin.

In some embodiments, the antibiotic is an aminoglycoside, selected from 1,2' -N-DL -isoseryl-3' ,4' -dideoxykanamycin B, 1,2' -N-DL-isoseryl-kanamycin B, 1,2' -N[(S)-4-amino-2-hydroxybutyryl]-3' ,4' -dideoxykanamycin B, 1,2' -N-[(S) -4-amino-2-hydroxybutyryq-kanamycin B, 1-N-(2-Aminobutanesulfonyl) kanamycin A, 1-N-(2-aminoethanesulfonyl)3,4' - dideoxyribostamycin, 1-N-(2-Aminoethanesulfonyl)3' -deoxyribostamycin, 1-N-(2-aminoethanesulfonyl)3' ,4' -dideoxykanamycin B, 1-N-(2-aminoethanesulfonyl)kanamycin A, 1-N-(2-aminoethanesulfonyl)kanamycin B, 1-N-(2-aminoethanesulfonyl)ribostamycin, 1-N-(2-aminopropanesulfonyl)3' -deoxykanamycin B, 1-N-(2-aminopropanesulfonyl)3' ,4' - dideoxykanamycin B, 1-N-(2-aminopropanesulfonyl)kanamycin A, 1-N -(2-aminopropanesulfonyl)kanamycin B, 1 -N-(L-4-amino-2-hydroxy -butyryl)2,' 3' -dideoxy-2' - fluorokanamycin A, 1-N-(L-4-amino-2-hydroxy -propionyl)2,' 3' -dideoxy-2' -fluorokanamycin A, 1-N-DL-3' ,4' -dideoxy -isoserylkanamycin B,1-N-DL-isoserylkanamycin, 1-N-DL-isoserylkanamycin B, 1-N[L-(-)-(alpha-hydroxy-gamma-aminobutyryl)]-XK-62-2, 2' ,3' - dideoxy-2' -fluorokanamycin A,2-hydroxygentamycin A3, 2-hydroxygentamycin B, 2-hydroxygentamycin B1, 2-hydroxygentamycin JI-20A, 2-hydroxygentamycin JI-20B, 3" -N-methyl-4" -C-methyl-3' ,4' -dodeoxy kanamycin A, 3" -N-methyl-4" -C-methyl-3' ,4' - dodeoxy kanamycin B, 3" -N-methyl-4" -C-methyl-3' ,4' -dodeoxy-6' -methyl kanamycin B, 3 ' ,4' -Dideoxy-3' -eno-ribostamycin, 3' ,4' -dideoxyneamine,3' ,4' -dideoxyribostamycin, 3' -deoxy-6' -N-methyl-kanamycin B,3' -deoxyneamine,3' -deoxyribostamycin, 3' - oxysaccharocin,3,3' -nepotrehalosadiamine, 3-demethoxy-2" -N -formimidoylistamycin B disulfate tetrahydrate, 3-demethoxyistamycin B,3-O-demethyl-2-N-formimidoylistamycin B, 3-O-demethylistamycin B,3-trehalosamine,4" ,6" -dideoxydibekacin, 4-N-glycyl-KA-6606VI, 5" - Amino-3' ,4' ,5" -trideoxy-butirosin A, 6" -deoxydibekacin,6' -epifortimicin A, 6-deoxy-neomycin (structure 6-deoxy-neomycin B),6-deoxy-neomycin B, 6-deoxy-neomycin C, 6-deoxy-paromomycin, acmimycin, AHB-3' ,4' -dideoxyribostamycin,AHB-3' -deoxykanamycin B, AHB-3' -deoxyneamine,AHB-3' -deoxyribostamycin,AHB-4" -6" -dideoxydibekacin, AHB-6" -deoxydibekacin,AHB-dideoxyneamine,AHB-kanamycin B, AHB -methyl-3' - deoxykanamycin B, amikacin, amikacin sulfate, apramycin, arbekacin, astromicin, astromicin sulfate, bekanamycin, bluensomycin, boholmycin, butirosin, butirosin B, catenulin, coumamidine gamma1, coumamidine gamma2,D,L-1-N-(alpha-hydroxy-beta-aminopropionyl) -XK-62-2, dactimicin,de-O-methyl-4-N-glycyl-KA-6606VI,de-O-methyl-KA -66061, de-O-methyl-KA-70381,destomycin A, destomycin B, di-N6' ,O3-demethylistamycin A, dibekacin, dibekacin sulfate, dihydrostreptomycin, dihydrostreptomycin sulfate, epi-formamidoylglycidylfortimicin B, epihygromycin, formimidoyl-istamycin A, formimidoyl-istamycin B, fortimicin B, fortimicin C, fortimicin D, fortimicin KE, fortimicin KF, fortimicin KG, fortimicin KG1 (stereoisomer KG1 /KG2), fortimicin KG2(stereoisomer KG1 /KG2), fortimicin KG3, framycetin, framycetin sulphate, gentamicin, gentamycin sulfate, globeomycin, hybrimycin A1, hybrimycin A2, hybrimycin B1, hybrimycin B2, hybrimycin C1, hybrimycin C2, hydroxystreptomycin, hygromycin, hygromycin B, isepamicin, isepamicin sulfate, istamycin, kanamycin, kanamycin sulphate, kasugamycin, lividomycin, marcomycin, micronomicin, micronomicin sulfate, mutamicin, myomycin, N-demethyl-7-O-demethylcelesticetin, demethylcelesticetin, methanesulfonic acid derivative of istamycin, nebramycin, nebramycin, neomycin, netilmicin, oligostatin, paromomycin, quintomycin, ribostamycin, saccharocin, seldomycin, sisomicin, sorbistin, spectinomycin, streptomycin, tobramycin, trehalosmaine, trestatin, validamycin, verdamycin, xylostasin, and zygomycin;

In some embodiments, the antibiotic is an ansa-type antibiotic selected from 21-hydroxy-25-demethyl-25-methylthioprotostreptovaricin, 3-methylthiorifamycin, ansamitocin, atropisostreptovaricin, awamycin, halomicin, maytansine, naphthomycin, rifabutin, rifamide, rifampicin, rifamycin, rifapentine, rifaximin, rubradirin, streptovaricin, and tolypomycin.

In some embodiments, the antibiotic is an anthraquinone selected from auramycin, cinerubin, ditrisarubicin, ditrisarubicin C, figaroic acid fragilomycin, minomycin, rabelomycin, rudolfomycin, and sulfurmycin.

In some embodiments, the antibiotic is an azole selected from azanidazole, bifonazole, butoconazol, chlormidazole, chlormidazole hydrochloride, cloconazole, cloconazole monohydrochloride, clotrimazol, dimetridazole, econazole, econazole nitrate, enilconazole, fenticonazole, fenticonazole nitrate, fezatione, fluconazole, flutrimazole, isoconazole, isoconazole nitrate, itraconazole, ketoconazole, lanoconazole, metronidazole, metronidazole benzoate, miconazole, miconazole nitrate, neticonazole, nimorazole, niridazole, omoconazol, ornidazole, oxiconazole, oxiconazole nitrate, propenidazole, secnidazol, sertaconazole, sertaconazole nitrate, sulconazole, sulconazole nitrate, tinidazole, tioconazole, and voriconazol.

In some embodiments, the antibiotic is a glycopeptide selected from acanthomycin, actaplanin, avoparcin, balhimycin, bleomycin B (copper bleomycin), chloroorienticin, chloropolysporin, demethylvancomycin, enduracidin, galacardin, guanidylfungin, hachimycin, demethylvancomycin, N -nonanoyl-teicoplanin, phleomycin, platomycin, ristocetin, staphylocidin, talisomycin, teicoplanin, vancomycin, victomycin, xylocandin, and zorbamycin.

In some embodiments, the antibiotic is a macrolide selected from acetylleucomycin, acetylkitasamycin, angolamycin, azithromycin, bafilomycin, brefeldin, carbomycin, chalcomycin, cirramycin, clarithromycin, concanamycin, deisovaleryl-niddamycin, demycinosyl-mycinamycin, Di-O -methyltiacumicidin, dirithromycin, erythromycin, erythromycin estolate, erythromycin ethyl succinate, erythromycin lactobionate, erythromycin stearate, flurithromycin, focusin, foromacidin, haterumalide, haterumalide, josamycin, josamycin ropionate, juvenimycin, juvenimycin, kitasamycin, ketotiacumicin, lankavacidin, lankavamycin, leucomycin, machecin, maridomycin, megalomicin, methylleucomycin, methymycin, midecamycin, miocamycin, mycaminosyltylactone, mycinomycin, neutramycin, niddamycin, nonactin, oleandomycin, phenylacetyldeltamycin, pamamycin, picromycin, rokitamycin, rosaramicin, roxithromycin, sedecamycin, shincomycin, spiramycin, swalpamycin, tacrolimus, telithromycin, tiacumicin, tilmicosin, treponemycin, troleandomycin, tylosin, and venturicidin.

In some embodiments, the antibiotic is a nucleoside selected from amicetin, angustmycin, azathymidine, blasticidin S, epiroprim, flucytosine, gougerotin, mildiomycin, nikkomycin, nucleocidin, oxanosine, oxanosine, puromycin, pyrazomycin, showdomycin, sinefungin, sparsogenin, spicamycin, tunicamycin, uracil polyoxin, and vengicide.

In some embodiments, the antibiotic is a peptide selected from actinomycin, aculeacin, alazopeptin, amfomycin, amythiamycin, antifungal from Zalerion arboricola, antrimycin, apid, apidaecin, aspartocin, auromomycin, bacileucin, bacillomycin, bacillopeptin, bacitracin, bagacidin, beminamycin, beta-alanyl-L-tyrosine, bottromycin, capreomycin, caspofungine, cepacidine, cerexin, cilofungin, circulin, colistin, cyclodepsipeptide, cytophagin, dactinomycin, daptomycin, decapeptide, desoxymulundocandin, echanomycin, echinocandin B, echinomycin, ecomycin, enniatin, etamycin, fabatin, ferrimycin, ferrimycin, ficellomycin, fluoronocathiacin, fusaricidin, gardimycin, gatavalin, globopeptin, glyphomycin, gramicidin, herbicolin, iomycin, iturin, iyomycin, izupeptin, janiemycin, janthinocin, jolipeptin, katanosin, killertoxin, lipopeptide antibiotic, lipopeptide from Zalerion sp., lysobactin, lysozyme, macromomycin, magainin, melittin, mersacidin, mikamycin, mureidomycin, mycoplanecin, mycosubtilin, neopeptifluorin, neoviridogrisein, netropsin, nisin, nocathiacin, nocathiacin 6-deoxyglycoside, nosiheptide, octapeptin, pacidamycin, pentadecapeptide, peptifluorin, permetin, phytoactin, phytostreptin, planothiocin, plusbacin, polcillin, polymyxin antibiotic complex, polymyxin B, polymyxin B1, polymyxin F, preneocarzinostatin, quinomycin, quinupristin-dalfopristin, safracin, salmycin, salmycin, salmycin, sandramycin, saramycetin, siomycin, sperabillin, sporamycin, a streptomyces compound, subtilin, teicoplanin aglycone, telomycin, thermothiocin, thiopeptin, thiostrepton, tridecaptin, tsushimycin, tuberactinomycin, tuberactinomycin, tyrothricin, valinomycin, viomycin, virginiamycin, and zervacin.

In some embodiments, the antibiotic is a polyene selected from amphotericin, amphotericin, aureofungin, ayfactin, azalomycin, blasticidin, candicidin, candicidin methyl ester, candimycin, candimycin methyl ester, chinopricin, filipin, flavofungin, fradicin, hamycin, hydropricin, levorin, lucensomycin, lucknomycin, mediocidin, mediocidin methyl ester, mepartricin, methylamphotericin, natamycin, niphimycin, nystatin, nystatin methyl ester, oxypricin, partricin, pentamycin, perimycin, pimaricin, primycin, proticin, rimocidin, sistomycosin, sorangicin, and trichomycin.

In some embodiments, the antibiotic is a polyether selected from 20-deoxy -epi-narasin, 20-deoxysalinomycin, carriomycin, dianemycin, dihydrolonomycin, etheromycin, ionomycin, iso-lasalocid, lasalocid, lenoremycin, lonomycin, lysocellin, monensin, narasin, oxolonomycin, a polycyclic ether antibiotic, and salinomycin.

In some embodiments, the antibiotic is a quinolone selected from alkyl -methylendioxy-4(1 H)-oxocinnoline-3-carboxylic acid, alatrofloxacin, cinoxacin, ciprofloxacin, ciprofloxacin hydrochloride, danofloxacin, dermofongin A, enoxacin, enrofloxacin, fleroxacin, flumequine, gatifloxacin, gemifloxacin, grepafloxacin, levofloxacin, lomefloxacin, lomefloxacin, hydrochloride, miloxacin, moxifloxacin, nadifloxacin, nalidixic acid, nifuroquine, norfloxacin, ofloxacin, orbifloxacin, oxolinic acid, pazufloxacine, pefloxacin, pefloxacin mesylate, pipemidic acid, piromidic acid, premafloxacin, rosoxacin, rufloxacin, sparfloxacin, temafloxacin, tosufloxacin, and trovafloxacin.

In some embodiments, the antibiotic is a steroid selected from aminosterol, asoosteroside, cladosporide, dihydrofusidic acid, dehydro-dihydrofusidic acid, dehydrofusidic acid, fusidic acid, and squalamine.

In some embodiments, the antibiotic is a sulfonamide selected from chloramine, dapsone, mafenide, phthalylsulfathiazole, succinylsulfathiazole, sulfabenzamide, sulfacetamide, sulfachlorpyridazine, sulfadiazine, sulfadiazine silver, sulfadicramide, sulfadimethoxine, sulfadoxine, sulfaguanidine, sulfalene, sulfamazone, sulfamerazine, sulfamethazine, sulfamethizole, sulfamethoxazole, sulfamethoxypyridazine, sulfamonomethoxine, sulfamoxol, sulfanilamide, sulfaperine, sulfaphenazol, sulfapyridine, sulfaquinoxaline, sulfasuccinamide, sulfathiazole, sulfathiourea, sulfatolamide, sulfatriazin, sulfisomidine, sulfisoxazole, sulfisoxazole acetyl, and sulfacarbamide.

In some embodiments, the antibiotic is a tetracycline selected from dihydrosteffimycin, demethyltetracycline, aclacinomycin, akrobomycin, baumycin, bromotetracycline, cetocyclin, chlortetracycline, clomocycline, daunorubicin, demeclocycline, doxorubicin, doxorubicin hydrochloride, doxycycline, lymecyclin, marcellomycin, meclocycline, meclocycline sulfosalicylate, methacycline, minocycline, minocycline hydrochloride, musettamycin, oxytetracycline, rhodirubin, rolitetracycline, rubomycin, serirubicin, steffimycin, and tetracycline.

In some embodiments, the antibiotic is a dicarboxylic acid selected from adipic acid, pimelic acid, suberic acid, azelaic acid, sebacic acid, 1,11-undecanedioic acid, 1,12-dodecanedioic acid, 1,13-tridecanedioic acid, and 1,14-tetradecanedioic acid.

In some embodiments, the antibiotic is an antibiotic metal or a metal ion, wherein the metal is selected from silver, copper, zinc, mercury, tin, lead, bismutin, cadmium, chromium, and gold.

In some embodiments, the antibiotic is a silver compound selected from silver acetate, silver benzoate, silver carbonate, silver iodate, silver iodide, silver lactate, silver laurate, silver nitrate, silver oxide, silver palmitate, silver protein, and silver sulfadiazine.

In some embodiments, the antibiotic is an oxidizing agent or a substance that releases free radicals or active oxygen, selected from oxygen, hydrogen peroxide, benzoyl peroxide, elemental halogen species, oxygenated halogen species, bleaching agents, perchlorite species, iodine, iodate, and benzoyl peroxide.

In some embodiments, the antibiotic is a cationic antimicrobial agent selected from quaternary ammonium compounds, alkyltrimethyl ammonium bromide, cetrimide, benzalkonium chloride, n-alkyldimethylbenzyl ammonium chloride, dialkylmethyl ammonium halide, and dialkylbenzyl ammonium halide;

In some embodiments, the antibiotic is a compound selected from chlorhexidine acetate, chlorhexidine gluconate and chlorhexidine hydrochloride, picloxydine, alexidine, polihexanide, chlorproguanil hydrochloride, proguanil hydrochloride, metformin hydrochloride, phenformin, and buformin hydrochloride.

In some embodiments, the antibiotic is an agent selected from abomycin, acetomycin, acetoxycycloheximide, acetylnanaomycin, an actinoplanessp. Compound, actinopyrone, aflastatin, albacarcin, albacarcin, albofungin, albofungin, alisamycin, alpha-R,S - methoxycarbonylbenzylmonate, altromycin, amicetin, amycin, amycin demanoyl compound, amycine, amycomycin, anandimycin, anisomycin, anthramycin, anti-syphilis imune substance, anti-tuberculosis immune substance, antibiotic from Eschericia coli, antibiotics from Streptomycesrefuineus, anticapsin, antimycin, aplasmomycin, aranorosin, aranorosinol, arugomycin, ascofuranone, ascomycin, ascosin, Aspergillus flavus antibiotic, asukamycin, aurantinin, an Aureolic acid antibiotic substance, aurodox, avilamycin, azidamfenicol, azidimycin, bacillaene, a Bacillus larvae antibiotic, bactobolin, benanomycin, benzanthrin, benzylmonate, bicozamycin, bravomicin, brodimoprim, butalactin, calcimycin, calvatic acid, candiplanecin, carumonam, carzinophilin, celesticetin, cepacin, cerulenin, cervinomycin, chartreusin, chloramphenicol, chloramphenicol palmitate, chloramphenicol succinate sodium, chlorflavonin, chlorobiocin, chlorocarcin, chromomycin, ciclopirox, ciclopirox olamine, citreamicin, cladosporin, clazamycin, clecarmycin, clindamycin, coliformin, collinomycin, copiamycin, corallopyronin, corynecandin, coumermycin, culpin, cuprimyxin, cyclamidomycin, cycloheximide, dactylomycin, danomycin, danubomycin, delaminomycin, demethoxyrapamycin, demethylscytophycin, dermadin, desdamethine, dexylosyl-benanomycin, pseudoaglycone, dihydromocimycin, dihydronancimycin, diumycin, dnacin, dorrigocin, dynemycin, dynemycin triacetate, ecteinascidin, efrotomycin, endomycin, ensanchomycin, equisetin, ericamycin, esperamicin, ethylmonate, everninomicin, feldamycin, flambamycin, flavensomycin, florfenicol, fluvomycin, fosfomycin, fosfonochlorin, fredericamycin, frenolicin, fumagillin, fumifungin, funginon, fusacandin, fusafungin, gelbecidine, glidobactin, grahamimycin, granaticin, griseofulvin, griseoviridin, grisonomycin, hayumicin, hayumicin, hazymicin, hedamycin, heneicomycin, heptelicid acid, holomycin, humidin, isohematinic acid, karnatakin, kazusamycin, kristenin, L - dihydrophenylalanine, a L-isoleucyl-L-2-amino-4-(4' -amino-2' , 5' -cyclohexadienyl) derivative, lanomycin, leinamycin, leptomycin, libanomycin, lincomycin, lomofungin, lysolipin, magnesidin, manumycin, melanomycin, methoxycarbonylmethylmonate, methoxycarbonylethylmonate, methoxycarbonylphenylmonate, methyl pseudomonate, methylmonate, microcin, mitomalcin, mocimycin, moenomycin, monoacetyl cladosporin, monomethyl cladosporin, mupirocin, mupirocin calcium, mycobacidin, myriocin, myxopyronin, pseudoaglycone, nanaomycin, nancimycin, nargenicin, neocarcinostatin, neoenactin, neothramycin, nifurtoinol, nocardicin, nogalamycin, novobiocin, octylmonate, olivomycin, orthosomycin, oudemansin, oxirapentyn, oxoglaucine methiodide, pactacin, pactamycin, papulacandin, paulomycin, phaeoramularia fungicide, phenelfamycin, phenyl, cerulenin, phenylmonate, pholipomycin, pirlimycin, pleuromutilin, a polylactone derivative, polynitroxin, polyoxin, porfiromycin, pradimicin, prenomycin, Prop-2-enylmonate, protomycin, Pseudomonas antibiotic, pseudomonic acid, purpuromycin, pyrinodemin, pyrrolnitrin, pyrrolomycin, amino, chloro pentenedioic acid, rapamycin, rebeccamycin, resistomycin, reuterin, reveromycin, rhizocticin, roridin, rubiflavin, naphthyridinomycin, saframycin, saphenamycin, sarkomycin, sarkomycin, sclopularin, selenomycin, siccanin, spartanamicin, spectinomycin, spongistatin, stravidin, streptolydigin, streptomycesarenae antibiotic complex, streptonigrin, streptothricins, streptovitacin, streptozotocine, a strobilurin derivative, stubomycin, sulfamethoxazol - trimethoprim, sakamycin, tejeramycin, terpentecin, tetrocarcin, thermorubin, thermozymocidin, thiamphenicol, thioaurin, thiolutin, thiomarinol, thiomarinol, tirandamycin, tolytoxin, trichodermin, trienomycin, trimethoprim, trioxacarcin, tyrissamycin, umbrinomycin, unphenelfamycin, urauchimycin, usnic acid, uredolysin, variotin, vermisporin, verrucarin, and analogs, salts and derivatives thereof.

In some embodiments, the antibiotic agent is selected from the group of aminoglycoside, ansamycin, carbacephem, carbapenem, cephalosporin, fosfomycin, glycopeptide, lincosamide, lipopeptide, macrolide, monobactam, nitrofuran, oxazolidinone, penicillin, quinolone, sulfonamide, and tetracycline.

In some embodiments, at least 1, 2, 3, 4, or 5 or more antibiotic agents are selected from the group of aminoglycoside, ansamycin, carbacephem, carbapenem, cephalosporin, fosfomycin, glycopeptide, lincosamide, lipopeptide, macrolide, monobactam, nitrofuran, oxazolidinone, penicillin, quinolone, sulfonamide, and tetracycline.

In some embodiments, the sample is exposed to two or more antimicrobial agents simultaneously. For instance, a sample of bacteria may comprise two or more antimicrobial agents. In some embodiments, a sample may comprise a beta-lactam antibiotic and a beta-lactamase inhibitor (BLI). In some embodiments, a sample comprises two or more antimicrobial agents, wherein the two or more antimicrobial agents are selected from the group of gentamicin, ciprofloxacin, cefazolin, ceftriaxone, cefepime, ampicillin, imipenem, trimethoprim, sulfamethoxazole, amikacin, nitrofurantoin, fosfomycin, piperacillin, tazobactam, amoxicillin, and clavulanate. In some embodiments, a sample comprises trimethoprim and sulfamethoxazole. In some embodiments, a sample comprises piperacillin and tazobactam. In some embodiments, a sample comprises amoxicillin and clavulanate.

In one embodiment the sample is exposed to at least 1, 2, 3, 4, or 5 or more antibiotic agents in the presence of Rnase. In one embodiment the Rnase is RNase A. In some embodiments, the RNase is RNase T1, RNase I, RNase VI, or RNase III. RNase V1 acts on double stranded (i.e., highly structured) RNA such as rRNA. RNase III specifically acts on pre-rRNA, not mature rRNA. Many RNases have some activity on rRNA, and those skilled in the art can select an appropriate RNase for selected embodiment.

In one embodiment, a wide range of RNase concentrations may be used in this method, from 0.01 to 10 micrograms RNase per milliliter growth medium.

In some embodiments, the RNase concentration used in this method is 0.02, 0.03, 0.04, 0.05, 0.06, 0.07, 0.08, 0.09, 0.1, 0.2, 0.3, 0.4, 0.5, 0.6, 0.7, 0.8, 0.9, 1, 1.5, 2, 2.5, 3, 3.5, 4, 4.5, 5, 5.5, 6, 6.5, 7, 7.5, 8, 8.5, 9 or 9.5 micrograms RNase per milliliter growth medium. In one embodiment the Rnase is RNase A. In some embodiments, the RNase is RNase T1, RNase I, RNase VI, or RNase III. RNase V1 acts on double stranded (i.e., highly structured) RNA such as rRNA. RNase III specifically acts on pre-rRNA, not mature rRNA.

In one embodiment, the RNase concentration used in this method is 1 microgram RNase per milliliter growth medium.

In some embodiments, the method further comprises adding sodium hydroxide (NaOH) to the growth medium prior to, or concurrently with, the measuring of step (c). In one example, the NaOH is used at a concentration of 1 M, for the purpose of releasing rRNA.

Also provided is a method for improving the sensitivity of an antibiotic susceptibility test. In one embodiment, the method comprises: (a) inoculating a specimen obtained from the sample into a growth medium in the presence a cell wall active antibiotic agent, wherein the growth medium comprises an RNase that hydrolyzes ribosomal RNA (rRNA). The method further comprises (b) inoculating a specimen obtained from the sample into a growth medium in the absence of the antibiotic agent, wherein the growth medium comprises an RNase that is enzymatically active against rRNA. Finally, the method comprises measuring the relative amounts of rRNA in the specimens of (a) and (b), and identifying the sample as susceptible to antibiotic treatment if the amount of rRNA measured in step (a) is reduced relative to the amount of rRNA measured in step (b). In one embodiment, the measuring of step (b) is implemented by using a known or expected amount of rRNA based on predictable conditions rather than actual testing.

In another embodiment, the invention provides a method for improving the sensitivity of an rRNA assay. In one embodiment, the method comprises: (a) obtaining a sample comprising living cells, and introducing into the sample an RNase that hydrolyzes rRNA, and then (b) inactivating the RNase prior to releasing rRNA from the living cells. The method further comprises measuring the amount of rRNA in the sample after releasing rRNA from the living cells. In one embodiment, the inactivating of step (b) is effected by contacting the sample with NaOH or other agent that inhibits or degrades RNase. In one example, 1 M NaOH is added to the medium prior to, or at the point of, processing the sample for measurement of rRNA. In one example, 25 microliters of 1 M NaOH is added to 50 microliters of sample comprising living cells. In this method, the ratio of 1 volume of 1 M NaOH to 2 volumes of sample is maintained across all total assay volumes.

For use in the methods described herein, representative examples of the sample include, but are not limited to, blood, plasma or serum, saliva, urine, cerebral spinal fluid, milk, cervical secretions, semen, tissue, cell cultures, and other bodily fluids or tissue specimens.

Disclosed herein are methods for determining the susceptibility of a microorganism such as bacteria to an antimicrobial agent such as an anibiotic agent. In some embodiments, the microorganism is susceptible to the antimicrobial agent if the quantity of nucleic acid molecules of the microorganism in the antimicrobial agent-free inoculate is more than the quantity of nucleic acid molecules of the microorganism in an inoculate comprising the microorganism and the antimicrobial agent. In some embodiments, the microorganism is not susceptible to the antimicrobial agent if the quantity of nucleic acid molecules of the microorganism in the antimicrobial agent-free inoculate is nearly equal, equal, or less than the quantity of nucleic acid molecules of the microorganism in an inoculate comprising the microorganism and the antimicrobial agent.

### EXAMPLES

The following examples are presented to illustrate the present invention and to assist one of ordinary skill in making and using the same. The examples are not intended in any way to otherwise limit the scope of the invention. The experiments below have been performed with Eubacterial/Universal Gram Negative probe sets. The sequences of the probes are included below. The sequence represented as SEQ ID No: 1 and the sequence represented as SEQ ID No: 4 depict the regions that hybridize to the ribosomal RNA while SEQ ID No: 2 binds the magnetic beads.

SEQ ID No: 3 represents a probe comprising two contiguously arranged sequences namely the rRNA target sequence hybridizing portion of the probe (i.e. SEQ ID No: 1) and the magnetic bead binding portion of the probe (i.e. SEQ ID No: 2).
EU GN Bead Cap 5'-GTTACGACTTCACCCCAG-3' (SEQ ID NO: 1)
5'-CATAATCAATTTCAACTTTCTACT-3' (SEQ ID NO: 2)
SEQ ID No: 3 - 5'-GTTACGACTTCACCCCAGCATAATCAATTTCAACTTTCTACT-3'
EU GN Bead Det 5' Biotin-GTTCCCCTACGGTTACCTT-3' (SEQ ID No: 4)

### Example 1: RNase Lowers Background and Improves Sensitivity of Microbial Detection and Antimicrobial Susceptibility Testing (AST)

We have discovered that addition of RNase to a sample lowers the limit of microbial detection and shortens the time required for AST. Rapid diagnostic tests to detect microbes and determine their antimicrobial susceptibility are urgently needed to guide antimicrobial therapy in patients with infections. Such tests are particularly important in patients at risk of infection with antibiotic resistant bacteria. This approach can be applied to tests that target rRNA to detect, identify, and perform AST on microbes. In a typical assay, levels of rRNA are measured by sample lysis and hybridization of rRNA with capture and detector probes. The detector probe is linked to a signaling molecule with enzymatic (eg. Horseradish peroxidase) or optical (eg. fluorescence, bioluminescence) features. Samples containing free rRNA not from living cells increases background, lowers the signal-to-noise ratio and increases the limit of detection. A low limit of detection improves the sensitivity of diagnostic tests designed to detect microbes. Sensitivity is also important for phenotypic AST based on the rRNA response to antibiotics. We treated Mueller-Hinton growth medium treated with or without 1 microgram per milliliter RNase for 30 minutes at 37°C with shaking prior to inoculation with bacteria. After incubation in a shaking incubator at 37°C, serial dilutions of th e cultures were performed followed by lysis and measurement of rRNA using a capture and detector probe pair. Cultures were simultaneously plated to measure CFU/ml. Critical limit (Lc) and limit of detection (Ld) were determined using a model based method, as previously described (Patel, M., et al., J Clin Microbiol 49:4293-6 (2011). PMID: 21940468.).

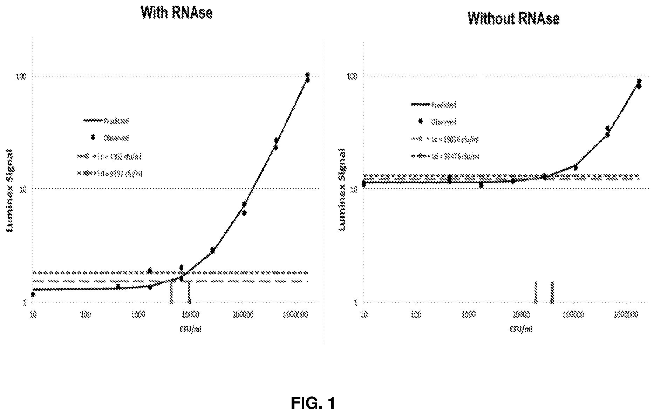

As shown in Figure 1, the background was much lower in the RNase-treated sample than in the untreated sample. As a result, the Lc and Ld were four-fold lower in the RNase-treated sample than in the untreated sample.

In the case of AST, rRNA is utilized as a surrogate marker for the phenotypic response to antibiotics. Bacterial suspensions are inoculated into growth medium with and without antibiotics followed by incubation at 37°C. At the conclusion of the incubation period, comparison of rRNA levels with and without antibiotics enables determination of whether the bacterial isolate is susceptible or resistant to the tested antibiotic. Faster AST would accelerate the time to therapy with antibiotics to which the patient's microbes are susceptible.

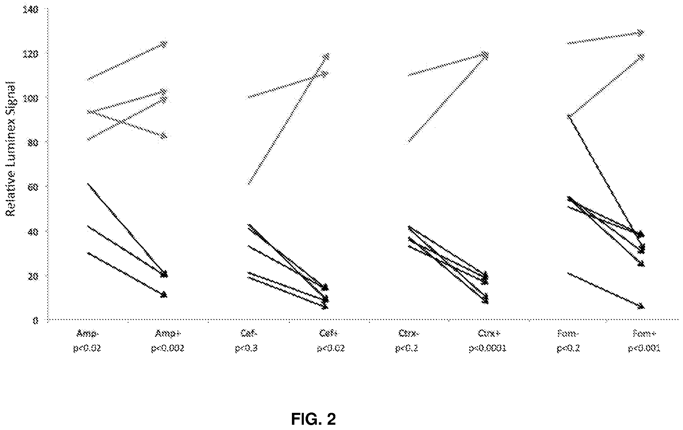

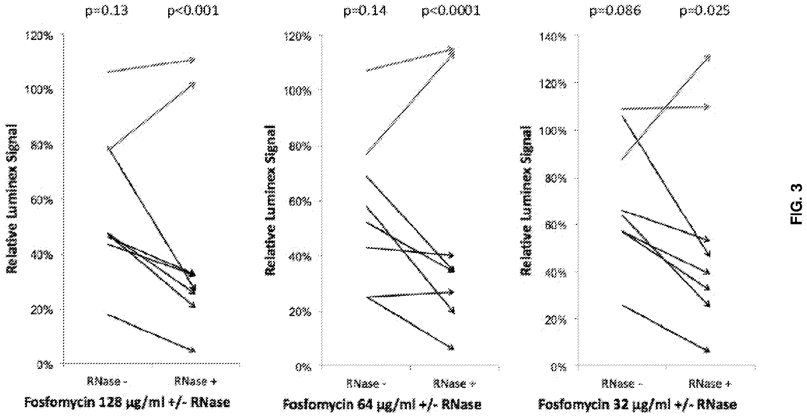

We performed AST on various bacterial isolates inoculated into wells of 96-well plates containing Mueller Hinton culture medium with and without 1 microgram per milliliter RNase added to the culture medium. RNase treatment of growth medium was performed as described above. The bacterial rRNA response to culture medium with and without the following cell wall active antibiotics was compared: ampicillin (Amp), cefazolin (Cef), ceftriaxone (Ctrx), and fosfomycin (Fom). After incubation in a shaking incubator at 37°C, lysis of the culture was performed followed by measurement of rRNA using a eubacterial capture and detector probe pair. The percent of rRNA in the wells containing antibiotic was compared to control wells without antibiotic. As shown in Figure 2, RNase dramatically increased the ability of this test to distinguish resistant (red arrows) from susceptible (black arrows) isolates. The results shown in Figure 2 were repeates with different concentrations of Fosfomycin: 128 µg/ml, 64 µg/ml and 32 µg/ml and the results are shown in Figure 3. Accordingly, when using different concentrations of Fosfomycin, RNase was able to dramatically increased the ability of this test to distinguish resistant (red arrows) from susceptible (black arrows) isolates.

### Example 2: Antimicrobial Susceptibility Testing (AST) Show RNase Advantage at 60 Minutes

This Example demonstrates comparison of antimicrobial susceptibility tests with and without 1 µg/ml RNase for *Escherichia coli* treated with four different antibiotics: Ampicillin (Amp), Cefazolin (Cef), Ceftriaxone (Ctrx), and Fosfomycin (Fos). The data show an advantage with RNase at both 60 and 90 minutes after inoculation with different antibiotics. Similar results have been observed for other Gram-negative bacteria such as *Klebsiella pneumoniae, Proteus mirabilis,* and *Pseudomonas aeruginosa* using the same conditions and RNase concentrations as for *E*. *coli.*

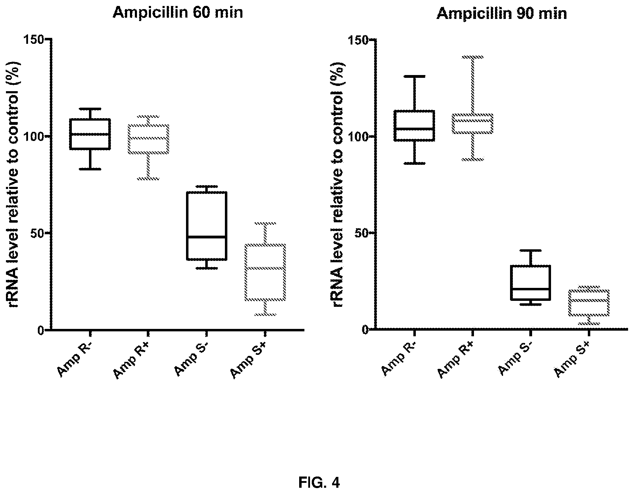

Susceptible (S) and resistant (R) *E. coli* isolates were incubated in growth medium with ampicillin for 60 or 90 minutes at 37°C with (+) an d without (-) RNase. Box and whisker plots of rRNA levels relative to control (growth medium without antibiotic) are shown in Figure 4. Susceptible isolates demonstrated a significant reduction in rRNA when RNase was added to the growth medium.

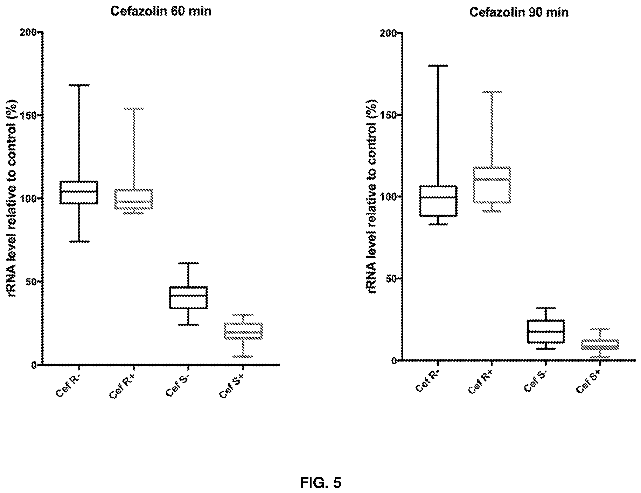

Susceptible (S) and resistant (R) *E. coli* isolates were incubated in growth medium with cefazolin for 60 or 90 minutes at 37°C with (+) and without (-) 1 µg/ml RNase. Box and whisker plots of rRNA levels relative to control (growth medium without antibiotic) are shown in Figure 5. Susceptible isolates demonstrated a significant reduction in rRNA when RNase was added to the growth medium.

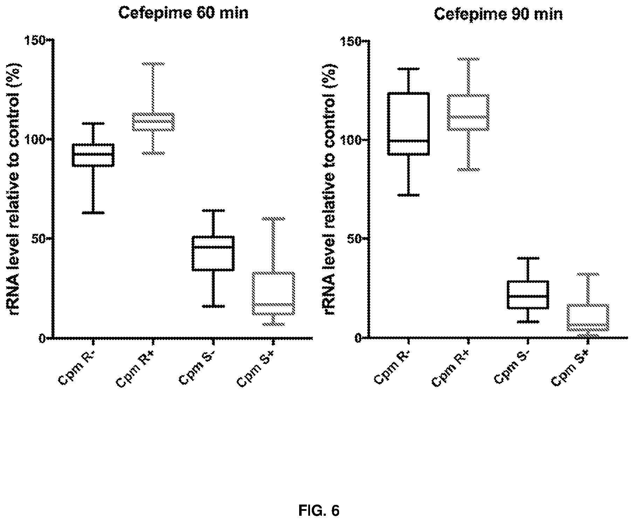

Susceptible (S) and resistant (R) *K. pneumoniae* isolates were incubated in growth medium with cefepime for 60 or 90 minutes at 37°C with (+) and without (-) 1 µg/ml RNase. Box and whisker plots of rRNA levels relative to control (growth medium without antibiotic) are shown in Figure 6. Susceptible isolates demonstrated a significant reduction in rRNA when RNase was added to the growth medium.

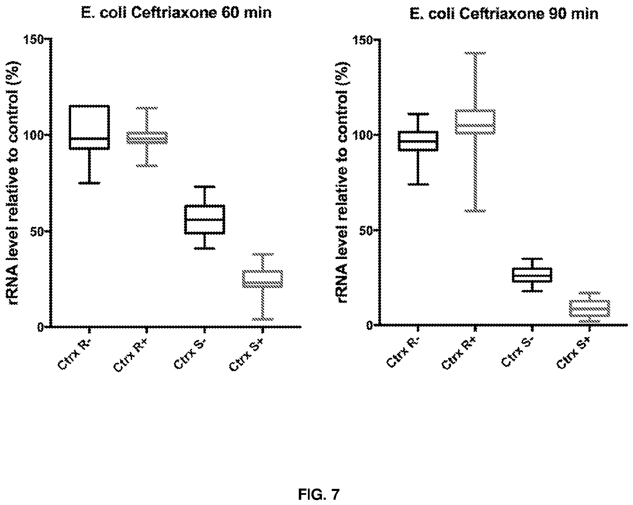

Susceptible (S) and resistant (R) *E. coli* isolates were incubated in growth medium with ceftriaxone for 60 or 90 minutes at 37°C with (+) a nd without (-) RNase. Box and whisker plots of rRNA levels relative to control (growth medium without antibiotic) are shown in Figure 7. Susceptible isolates demonstrated a significant reduction in rRNA when RNase was added to the growth medium.

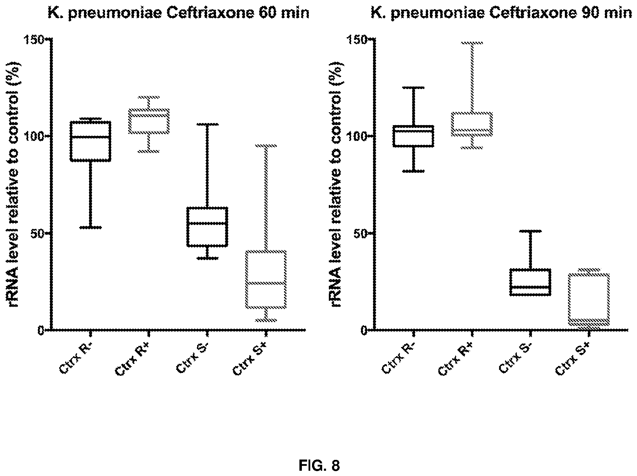

Susceptible (S) and resistant (R) *K. pneumoniae* isolates were incubated in growth medium with ceftriaxone for 60 or 90 minutes at 37°C with (+) a nd without (-) RNase. Box and whisker plots of rRNA levels relative to control (growth medium without antibiotic) are shown in Figure 8. Susceptible isolates demonstrated a significant reduction in rRNA when RNase was added to the growth medium.

Throughout this application various publications are referenced.

### References:

1. Halford, C., Gonzalez, R., Campuzano, S. Hu, B., Babbitt, J. T., Liu, J., Wang, J., Churchill, B. M., and Haake, D. A. "Rapid antimicrobial susceptibility testing by sensitive detection of precursor rRNA using a novel electrochemical biosensing platform," Antimicrob. Agents Chemother. 57(2):936-43 (2013). PMID: 23229486.
2. Patel, M., Gonzalez, R., Landaw, E., Lewinski, M., Churchill, B. M., and Haake, D. A. "Target specific capture enhances electrochemical detection of bacterial pathogens." J Clin Microbiol 49:4293-6 (2011). PMID: 21940468.

## Claims

1. A method for improving the sensitivity of an rRNA assay, the method comprising the steps of:
(a) introducing an RNase that hydrolyzes rRNA into a sample comprising living cells;
(b) inactivating the RNase prior to releasing rRNA from the living cells;
(c) measuring the amount of rRNA in the sample after releasing rRNA from the living cells.

2. The method of claim 1, wherein the inactivating of step (b) is effected by contacting the sample with NaOH.

3. A method for determining whether a sample of bacteria is susceptible to an antibiotic agent, the method comprising the steps of:
(a) inoculating a specimen obtained from the sample into a growth medium in the presence of a cell wall active antibiotic agent, wherein the growth medium comprises an RNase that hydrolyzes ribosomal RNA (rRNA);
(b) inoculating a specimen obtained from the sample into a growth medium in the absence of the antibiotic agent, wherein the growth medium comprises an RNase that is enzymatically active against rRNA;
(c) measuring the relative amounts of rRNA in the specimens of (a) and (b);
(d) identifying the sample as susceptible to antibiotic treatment if the amount of rRNA measured in step (a) is reduced relative to the amount of rRNA measured in step (b).

4. The method of claim 3, wherein the measuring of steps (a) and (b) comprises detection of specific hybridization of an oligonucleotide probe to the rRNA.

5. The method of claim 4, wherein the oligonucleotide probe is 10-50 nucleotides in length and hybridizes to the rRNA over the full length of a target sequence of the rRNA.

6. The method of claim 4, wherein the oligonucleotide probe is 25-30 nucleotides in length and hybridizes to the rRNA over the full length of a target sequence of the rRNA.

7. The method of claim 3, wherein the RNase is selected from the group of RNase A, RNase T1, RNase I, RNase VI or RNase III.

8. The method of claim 7, wherein the rRNA comprises at least one of bacterial rRNA and fungal rRNA.

9. The method of claim 3, wherein the antibiotic agent is fosfomycin or a beta lactam antibiotic.

10. The method of claim 3, further comprising adding sodium hydroxide (NaOH) to the growth medium prior to the measuring of step (c).

11. A method for improving the sensitivity of an antibiotic susceptibility test, the method comprising the steps of: (a) inoculating a specimen obtained from the sample into a growth medium in the presence of a cell wall active antibiotic agent, wherein the growth medium comprises an RNase that hydrolyzes ribosomal RNA (rRNA);
(b) inoculating a specimen obtained from the sample into a growth medium in the absence of the antibiotic agent, wherein the growth medium comprises an RNase that is enzymatically active against rRNA;
(c) measuring the relative amounts of rRNA in the specimens of (a) and (b);
(d) identifying the sample as susceptible to antibiotic treatment if the amount of rRNA measured in step (a) is reduced relative to the amount of rRNA measured in step (b).

12. The method of claim 3, wherein at least two antimicrobial agents are selected from the group of aminoglycoside, ansamycin, carbacephem, carbapenem, cephalosporin, fosfomycin, glycopeptide, lincosamide, lipopeptide, macrolide, monobactam, nitrofuran, oxazolidinone, penicillin, quinolone, sulfonamide, and tetracycline.

13. The method of claim 12, wherein the cephalosporin is selected from the group of first generation cephalosporin, second generation cephalosporin, third generation cephalosporin, fourth generation cephalosporin, and fifth generation cephalosporin.

14. The method of claim 3, wherein the antibiotic agent is selected from the group of gentamicin, ciprofloxacin, cefazolin, ceftriaxone, cefepime, ampicillin, imipenem, trimethoprim, sulfamethoxazole, amikacin, nitrofurantoin, fosfomycin, piperacillin, tazobactam, amoxicillin, and clavulanate.

15. The method of any of claims 3 to 14, wherein at least two antimicrobial agents are selected from the group of gentamicin, ciprofloxacin, cefazolin, ceftriaxone, cefepime, ampicillin, imipenem, trimethoprim, sulfamethoxazole, amikacin, nitrofurantoin, fosfomycin, piperacillin, tazobactam, amoxicillin, and clavulanate.

16. The method of any of claims 3 to 15, wherein at least one antimicrobial agent is a beta-lactamase inhibitor.

17. The method of claim 16, wherein the beta-lactamase inhibitor is selected from clavulanate, sulbactam, tazobactam, avibactam, relebactam, tebipenem, γ-methylidene Penem, and boron based transition state inhibitors.

18. The method of claim 16 or 17, wherein the beta-lactamase inhibitor is accompanied by a beta-lactam antibiotic.

19. The method according to anyone of claims 1 to 18, wherein the RNase is selected from the group of RNase A, RNase T1, RNase I, RNase VI or RNase III.

20. The method of any one of claims 1 to 19, wherein the concentration of RNase in the growth medium is from 0.01 to 10 micrograms per milliliter.

21. The method of claim 20, wherein the concentration of RNase is greater than 0.4 micrograms per milliliter.

22. The method of claim 20, wherein the concentration of RNase is at greater than 0.8 micrograms per milliliter.

23. The method of claim 20, wherein the concentration of RNase is at least 1 microgram per milliliter.

24. The method of any of claims 1 to 23, wherein the microorganism is a prokaryote.

25. The method of claim 24, wherein the prokaryote is a Gram positive bacteria.

## Patentansprüche

1. Verfahren zum Verbessern der Empfindlichkeit eines rRNA-Assays, wobei das Verfahren die folgenden Schritte umfasst:
(a) Einbringen einer RNase, die rRNA hydrolysiert, in eine Probe, die lebende Zellen umfasst;
(b) Inaktivieren der RNase vor der Freisetzung von rRNA aus den lebenden Zellen;
(c) Messen der Menge an rRNA in der Probe nach Freisetzung von rRNA aus den lebenden Zellen.

2. Verfahren nach Anspruch 1, wobei die Inaktivierung von Schritt (b) durch Inkontaktbringen der Probe mit NaOH bewirkt wird.

3. Verfahren zum Bestimmen, ob eine Probe von Bakterien gegenüber einem Antibiotika-Wirkstoff suszeptibel ist, wobei das Verfahren die folgenden Schritte umfasst:
(a) Inokulieren einer Musterprobe, die aus der Probe erhalten wird, in einem Wachstumsmedium in Gegenwart eines zellwandaktiven Antibiotika-Wirkstoffs, wobei das Wachstumsmedium eine RNase umfasst, die ribosomale RNA (rRNA) hydrolysiert;
(b) Inokulieren einer Musterprobe, die aus der Probe erhalten wird, in einem Wachstumsmedium in Abwesenheit des Antibiotika-Wirkstoffs, wobei das Wachstumsmedium eine RNase umfasst, die enzymatisch gegen rRNA aktiv ist;
(c) Messen der relativen Mengen an rRNA in den Musterproben von (a) und (b);
(d) Identifizieren der Probe als suszeptibel gegenüber der Antibiotika-Behandlung, wenn die in Schritt (a) gemessene Menge an rRNA relativ zu der in Schritt (b) gemessenen rRNA reduziert wird.

4. Verfahren nach Anspruch 3, wobei das Messen der Schritte (a) und (b) das Erfassen einer spezifischen Hybridisierung einer Oligonukleotidsonde an die rRNA umfasst.

5. Verfahren nach Anspruch 4, wobei die Oligonukleotidsonde 10-50 Nukleotide lang ist und über die gesamte Länge einer Targetsequenz der rRNA an der rRNA hybridisiert.

6. Verfahren nach Anspruch 4, wobei die Oligonukleotidsonde 25-30 Nukleotide lang ist und über die gesamte Länge einer Zielsequenz der rRNA an der rRNA hybridisiert.

7. Verfahren nach Anspruch 3, wobei die RNase aus der Gruppe der RNase A, RNase T1, RNase I, RNase VI oder RNase III ausgewählt ist.

8. Verfahren nach Anspruch 7, wobei die rRNA mindestens eine von bakterieller rRNA und mykotischer rRNA umfasst.

9. Verfahren nach Anspruch 3, wobei der Antibiotika-Wirkstoff Fosfomycin oder ein Beta-Lactam-Antibiotikum ist.

10. Verfahren nach Anspruch 3, ferner umfassend das Zugeben von Natriumhydroxid (NaOH) zu dem Wachstumsmedium vor der Messung von Schritt (c).

11. Verfahren zur Verbesserung der Empfindlichkeit eines Antibiotika-Suszeptibilitättests, wobei das Verfahren die folgenden Schritte umfasst:
(a) Inokulieren einer Musterprobe, die aus der Probe erhalten wird, in einem Wachstumsmedium in Gegenwart eines zellwandaktiven Antibiotika-Wirkstoffs, wobei das Wachstumsmedium eine RNase umfasst, die ribosomale RNA (rRNA) hydrolysiert;
(b) Inokulieren einer Musterprobe, die aus der Probe erhalten wird, in einem Wachstumsmedium in Abwesenheit des Antibiotika-Wirkstoffs, wobei das Wachstumsmedium eine RNase umfasst, die enzymatisch gegen rRNA aktiv ist;
(c) Messen der relativen Mengen an rRNA in den Musterproben von (a) und (b);
(d) Identifizieren der Probe als suszeptible gegenüber der Antibiotika-Behandlung, wenn die in Schritt (a) gemessene Menge an rRNA relativ zu der in Schritt (b) gemessenen rRNA reduziert wird.

12. Verfahren nach Anspruch 3, wobei mindestens zwei antimikrobielle Wirkstoffe ausgewählt sind aus der Gruppe von Aminoglykosid, Ansamycin, Carbacephem, Carbapenem, Cephalosporin, Fosfomycin, Glycopeptid, Linkosamid, Lipopeptid, Macrolid, Monobactam, Nitrofuran, Oxazolidinon, Penicillin, Quinolon, Sulfonamid und Tetracyclin.

13. Verfahren nach Anspruch 12, wobei das Cephalosporin ausgewählt ist aus der Gruppe der ersten Generation von Cephalosporin, zweiten Generation von Cephalosporin, dritten Generation von Cephalosporin, vierten Generation von Cephalosporin und fünften Generation von Cephalosporin.

14. Verfahren nach Anspruch 3, wobei der Antibiotika-Wirkstoff ausgewählt ist aus der Gruppe von Gentamicin, Ciprofloxacin, Cefazolin, Ceftriaxon, Cefepim, Ampicillin, Imipenem, Trimethoprim, Sulfamethoxazol, Amikacin, Nitrofurantoin, Fosfomycin, Piperacillin, Tazobactam, Amoxicillin, und Clavulanat.

15. Verfahren nach einem der Ansprüche 3 bis 14, wobei mindestens zwei antimikrobielle Wirkstoffe ausgewählt sind aus der Gruppe von Gentamicin, Ciprofloxacin, Cefazolin, Ceftriaxon, Cefepim, Ampicillin, Imipenem, Trimethoprim, Sulfamethoxazol, Amikacin, Nitrofurantoin, Fosfomycin, Piperacillin, Tazobactam, Amoxicillin, und Clavulanat.

16. Verfahren nach einem der Ansprüche 3 bis 15, wobei mindestens ein antimikrobieller Wirkstoff ein Beta-Lactamase-Inhibitor ist.

17. Verfahren nach Anspruch 16, wobei der Beta-Lactamase-Inhibitor ausgewählt ist aus Clavulanat, Sulbactam, Tazobactam, Avibactam, Relebactam, Tebipenem, γ-Methyliden-Penem, und Bor-basierten Übergangszustand-Inhibitoren.

18. Verfahren nach Anspruch 16 oder 17, wobei der Beta-Lactamase-Inhibitor von einem Beta-Lactam-Antibiotikum begleitet wird.

19. Verfahren nach einem der Ansprüche 1 bis 18, wobei die RNase aus der Gruppe der RNase A, RNase T1, RNase I, RNase VI oder RNase III ausgewählt ist.

20. Verfahren nach einem der Ansprüche 1 bis 19, wobei die Konzentration von RNase im Wachstumsmedium von 0,01 bis 10 Mikrogramm pro Milliliter beträgt.

21. Verfahren nach Anspruch 20, wobei die Konzentration von RNase größer als 0,4 Mikrogramm pro Milliliter beträgt.

22. Verfahren nach Anspruch 20, wobei die Konzentration von RNase mehr als 0,8 Mikrogramm pro Milliliter beträgt.

23. Verfahren nach Anspruch 20, wobei die Konzentration von RNase mindestens 1 Mikrogramm pro Milliliter beträgt.

24. Verfahren nach einem der Ansprüche 1 bis 23, wobei der Mikroorganismus ein Prokaryot ist.

25. Verfahren nach Anspruch 24, wobei der Prokaryot ein grampositives Bakterium ist.

## Revendications

1. Procédé pour améliorer la sensibilité d'un dosage d'ARNr, le procédé comprenant les étapes consistant à :
(a) introduire une ARNase qui hydrolyse de l'ARNr dans un échantillon comprenant des cellules vivantes ;
(b) inactiver l'ARNase avant de libérer de l'ARNr des cellules vivantes ;
(c) mesurer la quantité d'ARNr dans l'échantillon après libération d'ARNr des cellules vivantes.

2. Procédé selon la revendication 1, dans lequel l'inactivation de l'étape (b) est effectuée par mise en contact de l'échantillon avec du NaOH.

3. Procédé pour déterminer si un échantillon de bactéries est sensible à un agent antibiotique, le procédé comprenant les étapes consistant à :
(a) inoculer un spécimen obtenu à partir de l'échantillon dans un milieu de croissance en présence d'un agent antibiotique actif sur la paroi cellulaire, le milieu de croissance comprenant une ARNase qui hydrolyse de l'ARN ribosomique (ARNr) ;
(b) inoculer un spécimen obtenu à partir de l'échantillon dans un milieu de croissance en l'absence de l'agent antibiotique, le milieu de croissance comprenant une ARNase qui est enzymatiquement active vis-à-vis de l'ARNr ;
(c) mesurer les quantités relatives d'ARNr dans les spécimens de (a) et de (b) ;
(d) identifier l'échantillon comme étant sensible à un traitement antibiotique si la quantité d'ARNr mesurée à l'étape (a) est réduite par rapport à la quantité d'ARNr mesurée à l'étape (b).

4. Procédé selon la revendication 3, dans lequel la mesure des étapes (a) et (b) comprend la détection d'une hybridation spécifique d'une sonde oligonucléotidique à l'ARNr.

5. Procédé selon la revendication 4, dans lequel la sonde oligonucléotidique a une longueur de 10 à 50 nucléotides et s'hybride à l'ARNr sur la longueur complète d'une séquence cible de l'ARNr.

6. Procédé selon la revendication 4, dans lequel la sonde oligonucléotidique a une longueur de 25 à 30 nucléotides et s'hybride à l'ARNr sur la longueur complète d'une séquence cible de l'ARNr.

7. Procédé selon la revendication 3, dans lequel l'ARNase est choisie dans le groupe d'ARNase A, ARNase T1, ARNase I, ARNase VI ou ARNase III.

8. Procédé selon la revendication 7, dans lequel l'ARNr comprend au moins un parmi de l'ARNr bactérien et de l'ARNr fongique.

9. Procédé selon la revendication 3, dans lequel l'agent antibiotique est de la fosfomycine ou un antibiotique bêta lactame.

10. Procédé selon la revendication 3, comprenant en outre l'ajout d'hydroxyde de sodium (NaOH) au milieu de croissance avant la mesure de l'étape (c).

11. Procédé pour améliorer la sensibilité d'un test de sensibilité à un antibiotique, le procédé comprenant les étapes consistant à : (a) inoculer un spécimen obtenu à partir de l'échantillon dans un milieu de croissance en présence d'un agent antibiotique actif sur la paroi cellulaire, le milieu de croissance comprenant une ARNase qui hydrolyse de l'ARN ribosomique (ARNr) ;
(b) inoculer un spécimen obtenu à partir de l'échantillon dans un milieu de croissance en l'absence de l'agent antibiotique, le milieu de croissance comprenant une ARNase qui est enzymatiquement active vis-à-vis de l'ARNr ;
(c) mesurer les quantités relatives d'ARNr dans les spécimens de (a) et de (b) ;
(d) identifier l'échantillon comme étant sensible à un traitement antibiotique si la quantité d'ARNr mesurée à l'étape (a) est réduite par rapport à la quantité d'ARNr mesurée à l'étape (b).

12. Procédé selon la revendication 3, dans lequel au moins deux agents antimicrobiens sont choisis dans le groupe d'aminoglycoside, ansamycine, carbacéphème, carbapénème, céphalosporine, fosfomycine, glycopeptide, lincosamide, lipopeptide, macrolide, monobactame, nitrofurane, oxazolidinone, pénicilline, quinolone, sulfonamide et tétracycline.

13. Procédé selon la revendication 12, dans lequel la céphalosporine est choisie dans le groupe de céphalosporine de première génération, céphalosporine de deuxième génération, céphalosporine de troisième génération, céphalosporine de quatrième génération, et céphalosporine de cinquième génération.

14. Procédé selon la revendication 3, dans lequel l'agent antibiotique est choisi dans le groupe de gentamicine, ciprofloxacine, céfazoline, ceftriaxone, céfépime, ampicilline, imipénème, triméthoprime, sulfaméthoxazole, amikacine, nitrofurantoïne, fosfomycine, pipéracilline, tazobactam, amoxicilline, et clavulanate.

15. Procédé selon l'une quelconque des revendications 3 à 14, dans lequel au moins deux agents antimicrobiens sont choisis dans le groupe de gentamicine, ciprofloxacine, céfazoline, ceftriaxone, céfépime, ampicilline, imipénème, triméthoprime, sulfaméthoxazole, amikacine, nitrofurantoïne, fosfomycine, pipéracilline, tazobactam, amoxicilline, et clavulanate.

16. Procédé selon l'une quelconque des revendications 3 à 15, dans lequel au moins un agent antimicrobien est un inhibiteur de bêta-lactamase.

17. Procédé selon la revendication 16, dans lequel l'inhibiteur de bêta-lactamase est choisi parmi clavulanate, sulbactam, tazobactam, avibactam, rélébactam, tébipénème, γ-méthylidène pénème et inhibiteurs d'état de transition à base de bore.

18. Procédé selon la revendication 16 ou 17, dans lequel l'inhibiteur de bêta-lactamase est accompagné d'un antibiotique bêta-lactame.

19. Procédé selon l'une quelconque des revendications 1 à 18, dans lequel l'ARNase est choisie dans le groupe d'ARNase A, ARNase T1, ARNase I, ARNase VI ou ARNase III.

20. Procédé selon l'une quelconque des revendications 1 à 19, dans lequel la concentration en ARNase dans le milieu de croissance va de 0,01 à 10 microgrammes par millilitre.

21. Procédé selon la revendication 20, dans lequel la concentration en ARNase est supérieure à 0,4 microgramme par millilitre.

22. Procédé selon la revendication 20, dans lequel la concentration en ARNase est à plus de 0,8 microgramme par millilitre.

23. Procédé selon la revendication 20, dans lequel la concentration en ARNase est d'au moins 1 microgramme par millilitre.

24. Procédé selon l'une quelconque des revendications 1 à 23, dans lequel le micro-organisme est un procaryote.

25. Procédé selon la revendication 24, dans lequel le procaryote est une bactérie à Gram positif.
